(19) Europäisches Patentamt
European Patent Office
Office européen des brevets

(11) Veröffentlichungsnummer : **0 057 873**
**B1**

# (12) EUROPÄISCHE PATENTSCHRIFT

(45) Veröffentlichungstag der Patentschrift : **25.07.84**

(21) Anmeldenummer : **82100611.1**

(22) Anmeldetag : **29.01.82**

(51) Int. Cl.³ : **C 07 C 69/95**, C 07 C101/38, C 07 C101/68, C 07 C 67/313, C 07 C 99/00

## (54) Verfahren zur Herstellung von Dimethylsuccinylosuccinat, dessen Dinatriumsalz, Dianilinodihydroterephthalsäuren, deren Dimethylestern und Salzen sowie von Dianilinoterephthalsäuren, deren Dimethylestern und Salzen.

(30) Priorität : **10.02.81 DE 3104644**

(43) Veröffentlichungstag der Anmeldung : **18.08.82 Patentblatt 82/33**

(45) Bekanntmachung des Hinweises auf die Patenterteilung : **25.07.84 Patentblatt 84/30**

(84) Benannte Vertragsstaaten : **CH DE FR GB LI**

(56) Entgegenhaltungen :
**DE-B- 1 118 215**
**DE-B- 1 768 049**
**DE-C- 1 082 907**
**DE-C- 1 144 285**
**US-A- 2 803 644**
**US-A- 2 821 541**
**US-A- 3 024 268**

(73) Patentinhaber : **BAYER AG**
**Konzernverwaltung RP Patentabteilung**
**D-5090 Leverkusen 1 Bayerwerk (DE)**

(72) Erfinder : **Rolf, Meinhard, Dr.**
**Berta-von-Suttner-Strasse 24**
**D-5090 Leverkusen (DE)**
Erfinder : **Schütze, Detlef-Ingo, Dr.**
**Altenberger-Dom-Strasse 170**
**D-5060 Bergisch-Gladbach 2 (DE)**
Erfinder : **Neeff, Rütger, Dr.**
**Berta-von-Suttner-Strasse 22**
**D-5090 Leverkusen (DE)**
Erfinder : **Runzheimer, Hans-Volker, Dr.**
**In der Hildscheid 2**
**D-5068 Odenthal-Glöbusch (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Verfahren zur Herstellung von Dimethylsuccinylosuccinat, dessen Dinatriumsalz sowie von Dianilinodihydroterephthalsäuren, deren Dimethylestern und Salzen sowie von Dianilinoterephthalsäuren, deren Dimethylestern und Salzen ausgehend von dem nach dem neuen Verfahren hergestellten Dinatriumsalz von Dimethylsuccinylosuccinat ohne Zwischenisolierung. Die genannten Verbindungen, insbesondere Dianilinoterephthalsäuren, sind wichtige Zwischenprodukte zur Herstellung von wertvollen violetten oder roten Chinacridonpigmenten.

In der Literatur wurden bereits Verfahren zur Herstellung von Dimethylsuccinylosuccinat und Dianilinoterephthalsäuren oder -estern vorgeschlagen [s. z. B. Ann. *229*, 52 (1885) ; Ann. *404*, 272 (1914) ; US 2 803 644 ; US 3 024 268 ; FR 1 352 663 ; JP 50 019-738 ; JP 52 059-135 ; DE-AS 1 144 284 ; DE-AS 1 082 907 ; DE-AS 1 328 683 ; DE-AS 2 542 494 ; DE-AS 1 118 215 ; JP 9 108 036].

Keines der literaturbekannten Verfahren kann in der Praxis voll befriedigen. So werden Dianilinoterephthalsäuren fast immer ausgehend von isoliertem und getrocknetem Dialkylsuccinylosuccinat hergestellt. Die zwischengeschaltete Trocknung und Isolierung erfordern erheblichen Aufwand an Zeit, Apparaten, Arbeitskraft und Energie. Nach der DE-AS 1 082 907 wird zwar in einem Einstufenverfahren gearbeitet, jedoch fällt der Dianilinoterephthalsäureester in einem Lösungsmittel wie Dimethylformamid an, das bei der folgenden Verseifungsreaktion nicht inert ist.

Zur Herstellung von Dimethylsuccinylosuccinat empfehlen die meisten Literaturvorschriften die Verwendung von metallischem Natrium.

Für eine großtechnische Herstellung ist diese Arbeitsweise aber wegen der damit verbundenen Brand- und Explosionsgefahr nur mit aufwendigen und teuren Sicherheitsvorkehrungen durchführbar. Trotzdem kann dabei auf den Einsatz von metallischem Natrium nicht verzichtet werden, da bei Verwendung von Alkoholaten als Basen die Ausbeuten deutlich tiefer liegen (s. DE-AS 1 082 907, S. 1, Zeile 34-40).

Als Lösungsmittel dienen meist inerte Aromaten ; Alkohole sind zwar schon vorgeschlagen worden, wurden aber als « aus kinetischen Gründen ungeeignete Verdünnungsmittel » bezeichnet (DE-AS 1 082 907, Seite 1, Zeile 48/49), die zu geringeren Ausbeuten führen. Da bei der Kondensation von Bernsteinsäuredimethylester zu Dimethylsuccinylosuccinat 4 Mol Methanol frei werden, erhält man bei der Kondensation in Lösungsmitteln im Laufe der Reaktion immer Gemische dieser Lösungsmittel mit Methanol, deren Auftrennung oft zeitraubend und kostspielig ist.

Überraschenderweise wurde nun gefunden, daß sich Dimethylsuccinylosuccinat in Methanol als Lösungsmittel und ohne Einsatz von metallischem Natrium in guten Ausbeuten herstellen läßt.

Das neue Verfahren zur Herstellung von Dimethylsuccinylosuccinat oder dessen Dinatriumsalz ist dadurch gekennzeichnet, daß man Bernsteinsäuredimethylester in einer 35-45 gewichtsprozentigen Lösung von 120-180 % Natriummethylat in Methanol kondensiert.

Die Umsetzung verläuft gemäß

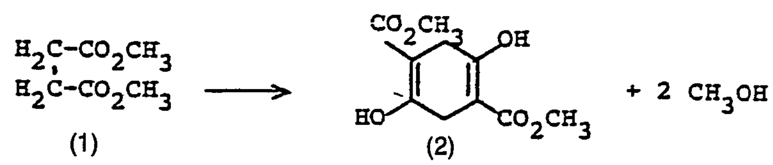


(1) (2)

Vorzugsweise wird das Verfahren folgendermaßen durchgeführt :

Bei 80-120 °C, vorzugsweise bei 90-100 °C, und gegebenenfalls unter Druck gibt man Bernsteinsäuredimethylester zu einer 35-45 gewichtsprozentigen Lösung von 120-180 %, vorzugsweise 140-160 % d. Th. Natriummethylat in Methanol, destilliert einen Teil des Methanols heraus und kocht gegebenenfalls noch 6-12 Stunden am Rückflußkühler. Gewünschtenfalls kann an dieser Stelle das Dinatriumsalz in an sich bekannter Weise isoliert werden. Durch einen Überschuß einer anorganischen oder organischen Säure. z. B. wäßrige Schwefelsäure Phosphorsäure, Salzsäure, Essigsäure oder Ameisensäure kann aus dem Dinatriumsalz, ohne oder nach Isolierung aus dem Rekationsgemisch Dimethylsuccinylosuccinat freigesetzt und durch Filtration in guter Ausbeute isoliert werden.

Das erfindungsgemäße Verfahren zur Herstellung von Dianilinodihydroterephthalsäuredimethylester der Formel

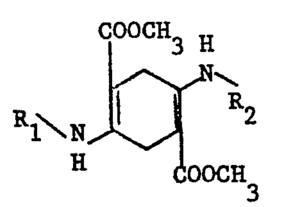

(I)

in der

$R_1 = R_2 =$ 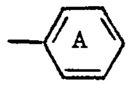 oder

$R_1 =$ 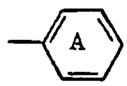 und $R_2 =$ 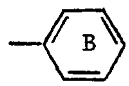 sind,

wobei die Ringe A und B durch 1 bis 4 Substituenten aus der Reihe $C_1$-$C_4$-Alkyl, Chlor, Fluor, $C_1$-$C_4$-Alkoxy, gegebenenfalls durch $C_1$-$C_4$-Alkyl mono- oder disubstituiertes Carbamoyl, Trifluormethyl, Carboxyl, Nitro substituiert sein können oder an sie ein aromatischer oder heteroaromatischer Ring ankondensiert sein kann, oder Dianilinodihydroterephthalsäuren bzw. deren Salze ist dadurch gekennzeichnet, daß man die durch Kondensation von Bernsteinsäuredimethylester in einer 35-45 gewichtsprozentigen Lösung von 120-180 % Natriummethylat in Methanol erhaltene Suspension des Dinatriumsalzes von Dimethylsuccinylosuccinat ansäuert, das freigesetzte Dimethylsuccinylosuccinat ohne Zwischenisolierung mit mindestens 2 Mol einer Verbindung der Formel

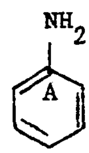 (II)

in der A die zu Formel (I) angegebene Bedeutung hat oder mit zusammen mindestens 2 Mol eines Gemisches einer Verbindung der Formel (II) und einer davon verschiedenen Verbindung der Formel (III)

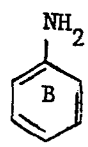 (III)

in der B die zu Formel (I) angegebene Bedeutung hat, oder mit Salzen der Amine (III) und/oder (II) mit anorganischen oder organischen Säuren kondensiert.

Die erhaltenen Dimethylester können in üblicher Weise im alkalischen Medium verseift und die Dicarbonsäuren durch Säurezusatz freigesetzt werden. Gewünschtenfalls können die Dimethylester, die durch die Verseifung entstandenen Alkalisalze und die Dicarbonsäuren in an sich bekannter Weise in Substanz isoliert werden. Diese Verbindungen können als Ausgangsmaterialien zur Synthese spezieller Chinacridone eingesetzt werden, indem man zunächst cyclisiert und anschließend zum Pigment oxidiert.

Vorzugsweise werden die Dimethylester der Formel (I) jedoch ohne Zwischenisolierung zu gegebenenfalls substituierten Dianilinoterephthalsäuren der Formel (IV)

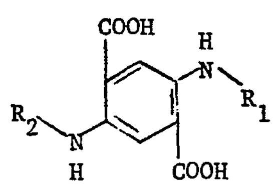 (IV)

in der $R_1$ und $R_2$ die zu Formel (I) angegebenen Bedeutungen haben bzw. zu Dimethylestern und Salzen der Dicarbonsäuren der Formel (IV) weiterverarbeitet.

Zu diesem Zweck wird die auf die oben angegebene Weise erhaltene Suspension der Ester der Dianilinodihydroterephthalsäure zu den Dimethylestern der Verbindungen der Formel (IV) oxidiert, die alkalisch zu den Salzen der Dianilinoterephthalsäuren der Formel (IV) verseift werden ; durch Säurezusatz lassen sich die freien Dicarbonsäuren (IV) freisetzen und in an sich bekannter Weise isolieren. Gewünschtenfalls können auch die Dimethylester und Salze der Verbindungen der Formel (IV) in üblicher Weise isoliert werden.

Als Amine der Formeln (II) und (III) werden vorzugsweise Anilin, p-Toluidin oder p-Chloranilin eingesetzt ; besonders bevorzugt setzt man mit nur einem Amin um.

In einer bevorzugten Ausführungsform wird die Suspension des Dinatriumsalzes von Dimethylsuccinylosuccinat z. B. mit Schwefelsäure, Salzsäure, Phosphorsäure, Essigsäure, Ameisensäure oder

Kohlensäure angesäuert, dann mit 110-200 % einer Verbindung der Formel (II) und (III), wobei diese Amine auch ganz oder teilweise in Form ihrer Salze mit den obengenannten Säuren vorliegen können, versetzt und bei 90-130 °C bis zur vollständigen Umsetzung gerührt. Die so erhaltene Suspension des Esters der Formel (I) wird dann ohne Isolierung alkalisch gestellt und bei 60-120 °C, gegebenenfalls unter Druck oxidiert. Bevorzugtes Oxidationsmittel ist Luft, aber auch Nitroaromaten wie m-Nitrobenzolsulfonsäure oder Nitroverbindungen der Formel

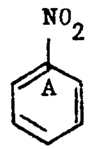

in welcher der Ring A' 1 bis 4 Substituenten aus der Reihe $C_1$-$C_4$-Alkyl, Chlor, Fluor, $C_1$-$C_4$-Alkoxy, gegebenenfalls durch $C_1$-$C_4$-Alkyl mono- oder disubstituiertes Carbamoyl, Trifluormethyl, Carboxy, Nitro tragen kann, oder an ihn ein aromatischer oder heterocyclischer Ring ankondensiert sein kann, ergeben sehr gute Resultate.

Als Sauerstoffüberträger bei der Luftoxidation werden vorzugsweise 0,5 bis 5 % (bezogen auf zu oxidierendes Material) Chinone, wie Anthrachinon, Phenanthrachinon, Naphthochinon und Chloranil, sowie deren Sulfon- und Carbonsäuren eingesetzt. Bevorzugt ist die Verwendung von Anthrachinonmono- und -disulfonsäuren, insbesondere von Anthrachinon-2-sulfonsäure.

Gleichzeitig mit der oxidation erfolgt in den meisten Fällen bereits die Verseifung zur Dicarbonsäure. Zur Vervollständigung kann man die Temperatur gegebenenfalls unter Druck auf 80-150 °C steigern und die Alkalimenge — bevorzugt Natriumhydroxid oder Kaliumhydroxid — erhöhen. Gegebenenfalls können zusätzlich Phasentransferkatalysatoren zugesetzt werden.

Aus der vorliegenden Suspension des Dialkalisalzes läßt sich die Dicarbonsäure durch einen Überschuß der obengenannten Säuren, gegebenenfalls nach Verdünnen mit Wasser und Klarfiltrieren, freisetzen und durch Filtration isolieren.

Das erfindungsgemäße Verfahren liefert die Dianilinoterephthalsäuren in guten Ausbeuten und in sehr hohen Reinheiten. Sie können ohne weitere Reinigung z. B. nach dem in US-PS 3 342 823 genannten Verfahren zu Chinacridon-Pigmenten umgesetzt werden.

Beispiel 1

Unter Stickstoff tropft man bei 95-100 °C zu 350 g 37 %iger Natriummethylat-Lösung in Methanol 234 g Bernsteinsäuredimethylester. Bei derselben Temperatur destilliert man dann 220 ml Methanol ab und kocht weitere 6 Stunden am Rückflußkühler. Anschließend wird durch Einrühren eines Gemisches von 1,2 kg Eis und 120 ml konz. Schwefelsäure angesäuert, bei Raumtemperatur abgesaugt und mit Wasser neutral gewaschen. nach dem Trocknen verbleiben 139 g (76 % d. Th.) Dimethylsuccinylosuccinat vom Schmelzpunkt 154-155 °C.

Beispiel 2

Unter Stickstoff tropft man bei 95-100 °C zu 350 g 37 %iger Natriummethylat-Lösung in Methanol 234 g Bernsteinsäuredimethylester. Bei derselben Temperatur destilliert man dann 220 ml Methanol ab und kocht weitere 6 Stunden am Rückflußkühler. Anschließend werden das methanolische Destillat, 80 g Methanol und 200 g Eisessig zugesetzt und homogen verrührt. Nach Zugabe von 143 g Anilin wird 2 Stunden unter Druck bei 105° gerührt, bei Raumtemperatur dann mit 3 g Anthrachinon-2-sulfonsäure und 500 g 30 %iger Natronlauge versetzt und bei ca. 70° unter gutem Rühren Luft durchgeblasen, bis sich eine Probe beim Verdünnen mit Wasser vollständig löst. Man setzt 2 l Wasser zu, filtriert heiß durch und säuert das Filtrat mit ca. 500 g konz. Schwefelsäure an. Nach Absaugen, Wäsche mit heißem Wasser und Trocknen bei 70 °C verbleiben 210 g (75 %) Dianilinoterephthalsäure der Formel

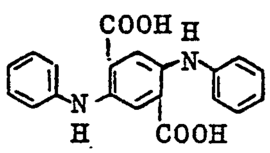

$C_{20}H_{16}N_2O_4$ (348.362)
ber. C : 68,96 H : 4,63 N : 8,04
gef. C : 69,0 H : 4,5 N : 8,1

Beispiel 2a

In gleich guter Qualität und Ausbeute erhält man die Dianilinoterephthalsäure, wenn man auf den

Zusatz von Anthrachinon-2-sulfonsäure und das Durchleiten von Luft verzichtet und stattdessen mit 132 g m-Nitrobenzolsulfonsäure (Na-Salz) oxidiert.

Beispiel 3

Nach dem in Beispiel 2 beschriebenen Verfahren erhält man bei Einsatz von 200 g p-Chloranilin anstelle von 143 g Anilin 240 g (72 % d. Th.) der Dichloranilinoterephthalsäure der Formel

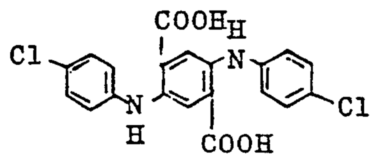

$C_{20}H_{14}Cl_2N_2O_4$ (417.252)
ber. C : 57,57 H : 3,38 N : 6,71
gef. C : 57,4 H : 3,4 N : 6,7

Beispiel 3a

In ähnlicher Ausbeute und Qualität erhält man die Dichlordianilinoterephthalsäure, wenn man die Oxidation statt mit Luft mit m-Nitrobenzolsulfonsäure durchführt.

Beispiel 4

Nach dem in Beispiel 2 beschriebenen Verfahren erhält man bei Einsatz von 165 g p-Toluidin anstelle von 143 g Anilin 226 g (75,1 %) der Ditoluidinoterephthalsäure der Formel

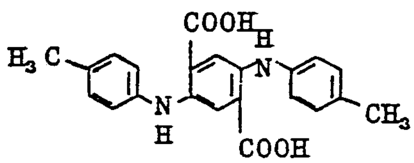

$C_{22}H_{20}N_2O_4$ (376.416)
ber. C : 70,20 H : 5,36 N : 7,44
gef. C : 70,2 H : 5,3 N : 7,5

Beispiel 4a

In ähnlicher Ausbeute und Qualität erhält man die Ditoluidinoterephthalsäure, wenn man die Oxidation statt mit Luft mit m-Nitrobenzolsulfonsäure durchführt.

Nach dem in Beispiel 1a genannten Verfahren erhält man unter Verwendung der in der folgenden Tabelle aufgeführen Aniline anstelle von Anilin die entsprechenden Dianilinoterephthalsäuren in sehr guten Reinheiten. Die Elementaranalysen der einzelnen Produkte stimmten gut mit den berechneten Werten überein.

| Beispiel | Anilin | Dianilinoterephtalsäure |
|---|---|---|
| 5 | 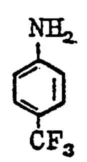 | 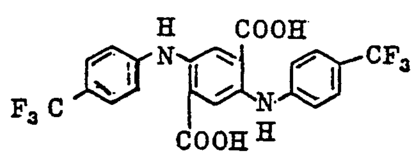 |
| 6 | 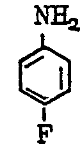 | 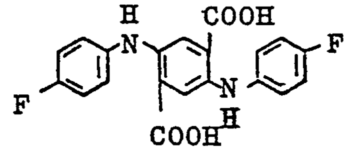 |
| 7 | 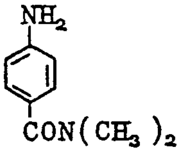 | 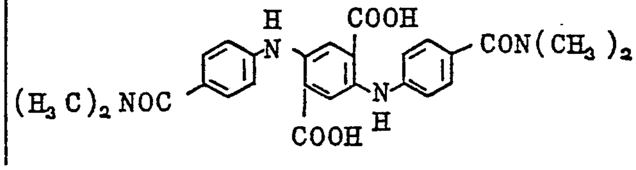 |

(Suite)

| Beispiel | Anilin | Dianilinoterephtalsäure |
| --- | --- | --- |
| 8 | 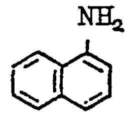 | 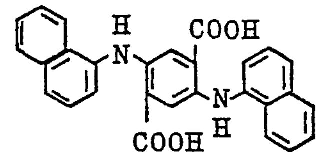 |
| 9 | 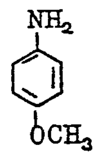 | 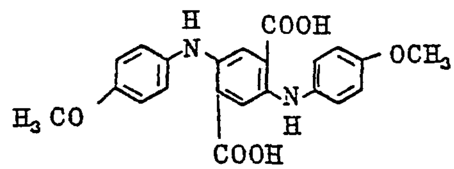 |
| 10 | 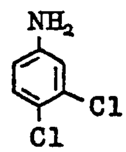 | 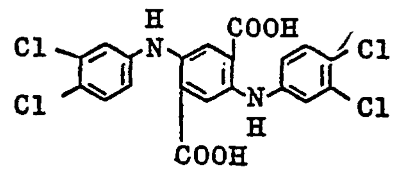 |
| 11 | 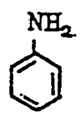 + 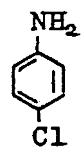 (1:1) | 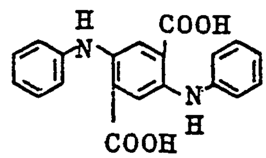 (ca. 25%) |
| | | 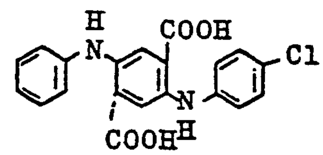 (ca. 50%) |
| | | 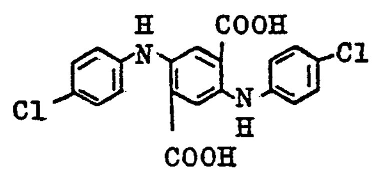 (ca. 25%) |

**Ansprüche**

1. Verfahren zur Herstellung von Dimethylsuccinylosuccinat oder dessen Dinatriumsalz, dadurch gekennzeichnet, daß man Bernsteinsäuredimethylester in einer 35-45 gewichtsprozentigen Lösung von 120-180 % Natriummethylat in methanol kondensiert und das Dinatriumsalz in an sich bekannter Weise isoliert oder durch eine Säure Dimethylsuccinylosuccinat freisetzt und in an sich bekannter Weise isoliert.

2. Verfahren zur Herstellung von Dianilinodihydroterephthalsäuredimethylestern der Formel (I),

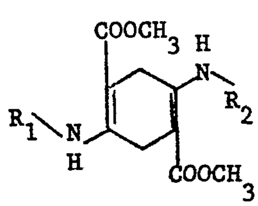

(I)

in der

$R_1 = R_2$ = 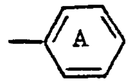 oder

$R_1$ = 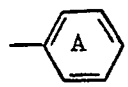 und $R_2$ = 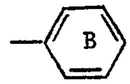 sind,

wobei die Ringe A und B durch 1 bis 4 Substituenten aus der Reihe $C_1$-$C_4$-Alkyl, Chlor, Fluor, $C_1$-$C_4$-Alkoxy, gegebenenfalls durch $C_1$-$C_4$-Alkyl mono- oder disubstituiertes Carbamoyl, Trifluormethyl, Carboxyl, Nitro substituiert sein können oder an sie ein aromatischer oder heteroaromatischer Ring ankondensiert sein kann, dadurch gekennzeichnet, daß man die durch Kondensation von Bernsteinsäuredimethylester gemäß Anspruch 1 erhaltene Suspension des Dinatriumsalzes von Dimethylsuccinylosuccinat ansäuert, das freigesetzte Dimethylsuccinylosuccinat ohne Zwischenisolierung mit mindestens zwei Mol einer Verbindung der Formel

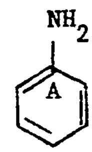 (II)

in der A die zu Formel (I) angegebene Bedeutung hat oder mit zusammen mindestens 2 Mol eines Gemisches einer Verbindung der Formel (II) und einer davon verschiedenen Verbindung der Formel (III)

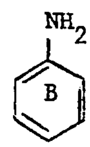 (III)

in der B die zu Formel (I) angegebene Bedeutung hat oder mit Salzen der Amine (III) und/oder (II), kondensiert und man die Dimethylester gegebenenfalls in an sich bekannter Weise isoliert.

3. Verfahren gemäß Anspruch 2, dadurch gekennzeichnet, daß man mit mindestens zwei Mol Anilin, p-Toluidin und/oder p-Chloranilin umsetzt.

4. Verfahren zur Herstellung von Dianilinodihydroterephthalsäuren oder deren Salzen, dadurch gekennzeichnet, daß man die gemäß den Verfahren der Ansprüche 2 und 3 erhaltenen Dimethylester ohne Zwischenisolierung alkalisch verseift, gegebenenfalls die Dicarbonsäuresalze in üblicher Weise isoliert oder aus ihnen ohne Zwischenisolierung die Dicarbonsäuren freisetzt und diese isoliert.

5. Verfahren zur Herstellung von Dianilinoterephthalsäuren der Formel

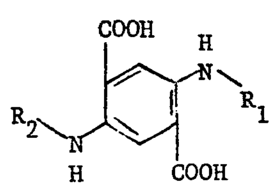 (IV)

in der $R_1$ und $R_2$ die zu Formel (I) angegebenen Bedeutungen haben, dadurch gekennzeichnet, daß man die nach den Verfahren der Ansprüche 2 und 3 erhaltene Suspension der dimethylester der Dianilinodihydroterephthalsäure oxidiert, die entstandenen Dimethylester der Dianilinoterephthalsäuren alkalisch zu den Salzen der Dianilinoterephthalsäuren verseift und durch Säurezusatz die freien Säuren der Formel (IV) freisetzt und isoliert.

6. Verfahren gemäß Anspruch 5, dadurch gekennzeichnet, daß man die Dimethylester der Dianilinodihydroterephthalsäuren mit Luft oxidiert.

7. Verfahren zur Herstellung der Dimethylester von Dianilinoterephthalsäuren der Formel (IV), dadurch gekennzeichnet, daß man diese im Verlaufe der Verfahren gemäß den Ansprüchen 5 und 6 in üblicher Weise isoliert.

8. Verfahren zur Herstellung der Salze von Dianilinoterephthalsäuren der Formel (IV), dadurch gekennzeichnet, daß man diese im Verlaufe der Verfahren.gemäß den Ansprüchen 5 und 6 in üblicher Weise isoliert.

**Claims**

1. Process for the preparation of dimethyl succinylosuccinate or the disodium salt thereof,

characterised in that dimethyl succinate is condensed in a 35-45 per cent by weight solution of 120-180 % of sodium methylate in methanol and the disodium salt is isolated in a manner which is in itself known, or dimethyl succinylosuccinate is liberated by means of an acid and is isolated in a manner which is in itself known.

2. Process for the preparation of dimethyl dianilinodihydroterephthalates of the formula (I),

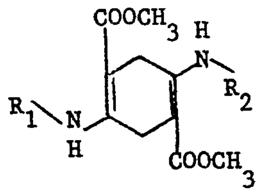

(I)

in which

$R_1 = R_2 =$ 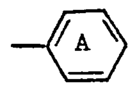 or

$R_1 =$ 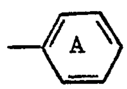 and $R_2 =$ 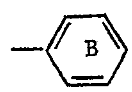

it being possible for the rings A and B to be substituted by 1 to 4 substituents from the series comprising $C_1$-$C_4$-alkyl, chlorine, fluorine, $C_1$-$C_4$-alkoxy, carbamoyl which is optionally monosubstituted or disubstituted by $C_1$-$C_4$-alkyl, trifluoromethyl, carboxyl or nitro, or it being possible for an aromatic or heteroaromatic ring to be fused to them, characterised in that the suspension of the disodium salt of dimethyl succinylosuccinate, which suspension is obtained by the condensation of dimethyl succinate according to Claim 1, is acidified, the liberated dimethyl succinylosuccinate is condensed, without intermediate isolation, with at least two mols of a compound of the formula

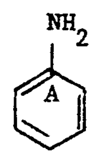

(II)

in which A has the meaning given under formula (I), or with, altogether, at least 2 mols of a mixture of a compound of the formula (II) and a compound, which differs therefrom, of the formula (III)

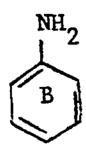

(III)

in which B has the meaning given under formula (I), or with salts of the amines (III) and/or (II), and the dimethyl esters are isolated, if appropriate, in a manner which is in itself known.

3. Process according to Claim 2, characterised in that the reaction is carried out using at least two mols of aniline, p-toluidine and/or p-chloroaniline.

4. Process for the preparation of dianilinodihydroterephthalic acids or the salts thereof, characterised in that the dimethyl esters obtained according to the processes of claims 2 and 3 are hydrolysed in an alkaline medium, without intermediate isolation, and, if appropriate, the dicarboxylic acid salts are isolated in the customary manner, or the dicarboxylic acids are liberated from them without intermediate isolation and these acids are isolated.

5. Process for the preparation of dianilinoterephthalic acids of the formula

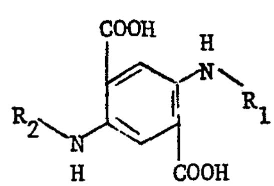

(IV)

in which $R_1$ and $R_2$ have the meanings given under formula (I), characterised in that the suspension of the dimethyl esters of dianilinodihydroterephthalic acid, which suspension is obtained according to the processes of Claims 2 and 3, is oxidised, the resulting dimethyl esters of dianilinoterephthalic acids are hydrolysed in an alkaline medium to the give the salts of the dianilinoterephthalic acids, and the free acids of the formula (IV) are liberated by the addition of acid and are isolated.

6. Process according to Claim 5, characterised in that the dimethyl esters of the dianilinodihydroterephthalic acids are oxidised using air.

7. Process for the preparation of the dimethyl esters of dianilinoterephthalic acids of the formula (IV), characterised in that these esters are isolated, in a customary manner, during the course of the processes according to Claims 5 and 6.

8. Process for the preparation of the salts of dianilinoterephthalic acids of the formula (IV), characterised in that these salts are isolated, in a customary manner, during the course of the processes according to Claims 5 and 6.

## Revendications

1. Procédé de préparation du succinylosuccinate de diméthyle ou de son sel disodique, caractérisé en ce que l'on condense le succinate de diméthyle dans une solution, à une concentration de 35 à 45 % en poids, de 120 à 180 % de méthylate de sodium dans le méthanol et on isole le sel disodique de manière connue en soi ou on libère le succinylosuccinate de diméthyle par un acide et on l'isole de manière connue en soi.

2. Procédé de préparation d'esters diméthyles d'acides dianilinodihydrotéréphtaliques de formule I

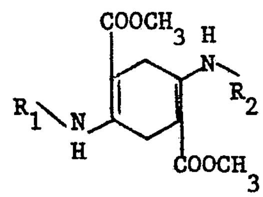

(I)

dans laquelle

$R_1 = R_2 =$ 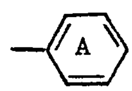 ou

$R_1 =$ 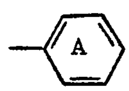 et $R_2 =$ 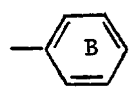

dans laquelle les noyaux A et B peuvent porter 1 à 4 substituants de la classe des groupes alkyle en $C_1$-$C_4$, du chlore, du fluor, des groupes alcoxy en $C_1$-$C_4$, du groupe carbamoyle éventuellement mono- ou di-substitué par des groupes alkyle en $C_1$-$C_4$, des groupes trifluorométhyle, carboxyle, nitro, ou être condensés sur un noyau aromatique ou hétéroaromatique, caractérisé en ce que l'on acidifie la suspension du sel disodique du succinylosuccinate de diméthyle obtenue par condensation du succinate de diméthyle selon la revendication 1, on condense le succinylosuccinate de diméthyle libéré, sans l'isoler au préalable, avec au moins 2 moles d'un composé de formule

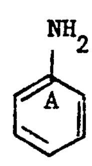

(II)

dans laquelle A a la signification indiquée en référence à la formule I, ou avec l'ensemble au moins 2 moles d'un mélange d'un composé de formule II et d'un composé différent de formule III

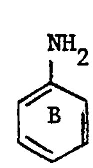

(III)

dans laquelle B a la signification indiquée en référence à la formule I, ou avec des sels des amines III et/ou II, et on isole les esters diméthyliques éventuellement de manière connue en soi.

3. Procédé selon la revendication 2, caractérisé en ce que l'on fait réagir avec au moins 2 moles d'aniline, de p-toluidine et/ou de p-chloraniline.

4. Procédé de préparation d'acides dianilinodihydrotéréphtaliques ou de leurs sels, caractérisé en ce que l'on soumet les esters diméthyliques obtenus par les procédés des revendications 2 et 3, sans les isoler au préalable, à une saponification alcaline, on isole éventuellement les sels d'acides dicarboxyliques de la manière habituelle ou on libère à partir de ces sels, sans les isoler au préalable, les acides dicarboxyliques qu'on isole ensuite.

5. Procédé de préparation des acides dianilinotéréphtaliques de formule

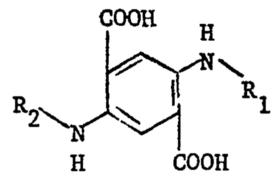

(IV)

dans laquelle $R_1$ et $R_2$ ont les significations indiquées en référence à la formule I, caractérisé en ce que l'on oxyde la suspension des esters diméthyliques de l'acide dianilinodihydrotéréphtalique obtenue par les procédés des revendications 2 et 3, on convertit les esters diméthyliques d'acides dianilinotéréphtaliques obtenus, par saponification alcaline, en les sels des acides dianilinotéréphtaliques et on libère et isole les acides libres de formule IV par addition d'un acide.

6. Procédé selon la revendication 5, caractérisé en ce que l'on oxyde les esters diméthyliques des acides dianilinodihydrotéréphtaliques à l'aide de l'air.

7. Procédé de préparation des esters diméthyliques des acides dianilinotéréphtaliques de formule IV, caractérisé en ce que l'on isole ces esters diméthyliques dans le cours de l'exécution des procédés selon les revendications 5 et 6 de la manière habituelle.

8. Procédé de préparation des sels des acides dianilinotéréphtaliques de formule IV, caractérisé en ce que l'on isole ces sels de la manière habituelle dans le cours de l'exécution des procédés selon les revendications 5 et 6.